Europäisches Patentamt

European Patent Office

Office européen des brevets

Publication number: **0 317 070**

**A2**

## EUROPEAN PATENT APPLICATION

Application number: 88309345.2

Date of filing: 07.10.88

Int. Cl.⁴: **C12Q 1/28** , //C12Q1/26, C12Q1/60

Priority: **08.10.87 US 106745**

Date of publication of application: **24.05.89 Bulletin 89/21**

Designated Contracting States: **AT BE CH DE ES FR GB GR IT LI LU NL SE**

Applicant: ENZYMATICS, INC.
355, Business Center Drive
Horsham, PA 19044(US)

Inventor: Palmer, John L.
150 N. Evergreen Avenue, D-3
Philadelphia, PA 19118(US)
Inventor: Timmerman, Marsha W.
2860 Reading Road
Allentown, PA 18104(US)

Representative: Bassett, Richard Simon et al
ERIC POTTER & CLARKSON 14 Oxford Street
Nottingham NG1 5BP(GB)

Digital colorimetric quantitative assay system.

Digital colorimetric quantative assay system, method and devices are disolosed for the accurate measurement of, by colour change, instead of intensity, the concentration of hydrogen peroxide, which is used to determine the fluid concentration of commercial medical and industrial substances that react with oxidase enzymes to produce hydrogen peroxide.

The assay comprises a chromogen, capable of reducing hydrogen peroxide and changing colour, and a reductant with larger reduction potential than that of the chromogen. The reductant prevents accumulation of oxidised chromogen so that a threshold colour change is achieved. The reductant is air stable and may be disulphate, a water-soluble alkali metal salt of ferrocyanide, or NADP(H). The reduction of the peroxide or the oxidised chromogen may be promoted by a catalyst, such as NAD(P)H peroxidase, diaphorase or an organic polycyclic compound.

EP 0 317 070 A2

# DIGITAL COLORIMETRIC QUANTITATIVE ASSAY SYSTEM

## BACKGROUND OF THE INVENTION

### 1. Field of the Invention

This invention relates to a colorimetric quantitative analysis of concentrations of biological molecules or organic compounds that produce hydrogen peroxide as a product of enzyme oxidation. In addition, this invention also relates to the direct colorimetric quantitative analysis of hydrogen peroxide.

This invention improves over the prior art the accuracy by which the concentration of analyte in solution may be measured and allows the measurement of higher concentrations of hydrogen peroxide and the analytes that react to produce hydrogen peroxide than heretofore possible. By making use of an "on-off" color change instead of color intensity to determine analyte concentration, the direct determination of the concentration of a wide variety of medical and industrial substances is made possible. Because this invention does not rely upon the intensity of color change to determine concentration, the inaccuracies attendant with such colorimetric systems of quantitative analysis are not found in this invention. Instead, accurate measurement is made possible of high analyte concentrations without pre-measurement dilution, heretofore unknown in the prior art.

### 2. Brief Description of the Invention

The invention provides an improved system, devices and method for the colorimetric quantitative analysis of concentrations of hydrogen peroxide and the biological molecules and organic compounds that are oxidized by substrate-specific oxidase enzymes to produce it. The system, devices and method use an oxidase enzyme specific for the organic or biomolecular substrate, the concentration of which is to be measured, which enzyme reacts with the substrate to produce hydrogen peroxide; a chromogen that is a reductant of hydrogen peroxide, which chromogen generates color when oxidized by hydrogen peroxide; and an air stable first reductant of hydrogen peroxide that has a larger reduction potential than that of the chromogen, which first reductant is irreversibly and preferentially oxidized by hydrogen peroxide over the chromogen but generates no color, at least not in the color range in which the chromogen generates color. A catalyst may also be employed to promote the oxidation of either the first reductant or the chromogen by hydrogen peroxide.

The invention also provides for the measurement of the concentration of an organic compound, generally in a biological fluid sample, which is oxidized by a substrate-specific oxidase enzyme to produce hydrogen peroxide. In accordance with the process of this invention, the air-stable first reductant of hydrogen peroxide is oxidized by both the hydrogen peroxide and any chromogen that may have been oxidized by the hydrogen peroxide so that for each mole of hydrogen peroxide produced there is produced an equivalence of color less than the equivalence of hydrogen peroxide. A catalyst may be used to promote their reactions.

It is a unique feature of the invention that there is generated less than one equivalent of colored dye from the chromogen per mole of hydrogen peroxide. The larger reduction potential of the air-stable first hydrogen peroxide reductant delays any detectable color change by the chromogen until the air-stable first hydrogen peroxide reductant has been consumed. Therefore, no color change occurs if the concentration of hydrogen peroxide or biomolecular or organic substrate to be measured is less then a pre-determined level corresponding to the con centration of the air-stable hydrogen peroxide reductant. This permits the direct quantitative measurement of analyte concentration independent of color change intensity.

The invention provides a system, device and method using a combination of a plurality or regions of various concentrations of said first air-stable hydrogen peroxide reductant, which system, device and method, when reacted with unknown concentrations of analyte to be measured, will change color in those regions with concentrations of air-stable hydrogen peroxide reductant corresponding to levels of substrate less than that contained in the unknown. Correspondingly, those regions with concentrations of said first air-stable hydrogen peroxide reductant corresponding to levels of analyte greater than that contained in the unknown will not change color. The concentration of analyte contained in the unknown will be between the level corresponding to the largest concentration of said first air-stable hydrogen peroxide reductant that resulted in a color change and the level corresponding to the lowest concentration of said first air-stable hydrogen peroxide reductant that did not result in a color change. The smaller the increment between levels of said first air-stable hydrogen peroxide reductant in the plurality of regions, the greater the accuracy of the measurement of the unknown quantity of analyte.

Thus, the invention provides for the measurement without dilution of concentrations of commercially important medical and industrial analytes such as alcohol, cholesterol, glucose, lactic acid, bilirubin and other organic compounds that react with substrate-specific oxidase enzymes to produce hydrogen peroxide.

The invention also provides for oxygen porous polymeric assay devices, the units of which can be commercially mass produced, yet still accurately absorb a set and reproducible quantity of liquid sample to be assayed.

The invention is operative in two different ways to generate the color corresponding to the amount of material sought to be determined: by the development of color (from colorless to color) or by the reduction of color from a high color intensity to a lower color intensity within the visible and readable range. Generically therefore, the method of the invention relates and refers to "color change".

## 3. Brief Description of the Prior Art

The qualitative and quantitative measurement of medically and commercially important biological molecules by way of colorimetric assay of compounds produced by the reaction of the molecules with substate-specific enzymes is well known in both the scientific and patent literature. The earlier filed patent applications referred to above describe many such systems.

EP Application No. 8731089.5 (published as EP 279 988) and much of the prior art referred to therein make use of an enzyme called diaphorase, which is the generic name for an enzyme that catalyzes the NAD(P)H reduction of dyes or chromogens (prodyes) to produce color changes. One enzyme that has been well studied is the lipoyl dehydrogenase subunit of pyruvate dehydrogenase and alpha-ketoglutarate dehydrogenase (V. Massay, Biochem. Biophys. Acta., 37 314-322 [1960]). Massay studied the substrate specificity of lipoyl dehydrogenase and showed that the enzyme reduces a wide variety of lipoic acid derivatives, for example: DL-lipoic acid, DL-lipoyl glycine, DL-lipoyl-beta-alanine, DL-lipoyl glycylglycine, DL-carboethoxy lipoanilide, DL-lipoanilide, and DL-lipoamide, in addition to potassium ferricyanide. Diaphorase and lipoyl dehydrogenase have been reviewed (U. Schmidt, P. Graffen, K. Altland & H.W. Goedde, Advances in Enzymology, 32, 423-469 (1969) and C.H. Williams, The Enzymes, 13 106-219 (1976)).

These publications are incorporated herein by reference especially for (but not only for) their disclosure of lipoic acid derivatives or compounds suitable for use in the invention and for the diaphorases.

The use of diaphorase to produce changes in visible color is widely discussed in the literature. Indeed, one of the first assays for this enzyme involved the reduction of 2,6-dichlorophenolindophenol (DCPIP) by NADH. DCPIP is a dye, while reduced DCPIP is colorless. Boethling and Weaver (R.S. Boethling and T.L. Weaver, Clin. Chem., 25, 2040-2042 [1979]) report a diaphorase assay which utilizes the prodye, thiazolyl blue tetrazolium bromide. Upon reduction, this colorless molecule is converted into a colored formazan which has a maximum absorbence at 560 nm.

The tetrazolium salt 3-p-nitrophenyl-2-iodophenyl-5phenyltetrazolium (INT) and diaphorase were used in production of an automated assay for urinary lactate dehydrogenase (N.J. Hella and S. Weinhouse, Anal. Biochem., 13, 322-325 [1965]), and in the development of a single-step assay for serum lacate dehydrogenase (C.C. Allain, D.P. Henson, M.K. Nadel, and A.J. Knoblesdroff, Clin. Chem., 19. 223-227 [1973]). The colorimetry of diaphorase and the preparation of clinical chemical reagents has been discussed (F.J. Gella, M.T. Olivella, F. Pegueroles, and J. Gener., Clin. Chem., 27, 1686-1689 [1981]).

The use of diaphorase in colorimetric assays has also been discussed in the patent literature. U.S. Patent 4,556,634 describes the use of formic acid lower alkyl esters to stop a reaction containing dehydrogenase, diaphorase, NAD(P), and tetrazolium salt. In this patent, the amount of dehydrogenase is inferred from the amount of formazan dye produced in a given, carefully measured time.

U.S. Patent 4,427,771 discloses an assay method for amylase activity and a method of producing maltose dehydrogenase for use therein. In this patent, a sample of serum, saliva or urine is pretreated with alphaglucosidase, hexokinase, and ATP to remove glucose and maltose. A glucose polymer is added to the sample, along with maltose dehydrogenase, NAD or NADP, and diaphorase and a tetrazolium salt or phenazine methosulfate and a tetrazolium salt. The amylase breaks down the glucose polymer to form maltose. The maltose is oxidized by maltose dehydrogenase to form NADH or NAD(P)H, which then reacts with the tetrazolium salt to produce a colored dye.

U.S. Patent 4,351,899 describes the use of a test surface containing the dried residue resulting from the impregnation of the surface with a tetrazolium salt, NAD, a dehydrogenase, and an electron carrier. In this patent. diaphorase is not used. Instead, the electron carrier meldola blue is used to catalyze the transfer of electrons from NADH to the tetrazolium salt. Meldola blue is also used as an electron carrier in U.S. Patent 4.254.222. which discusses the assay of lactic acid and beta-hydroxy butyrate via the dehydrogenases lactic dehydrogenase and beta-hydroxybutyrate dehydrogenase.

U.S. Patent 4.271.265 describes the use of diaphorase or electron transfer agents, tetrazolium salts. and NADP in the assay of glutamate-oxalacetate transaminase and glutamate-pyruvate transaminase.

U.S. Patent 4.247.633 describes the production of a dried, all-in-one reagent for the assay of creatine phosphokinase. This dried reagent contains: ADP, creatine phosphate, magnesium ions. glucose, hexokinase, NAD or NADP, INT, diaphorase, buffer, reduced glutathione, and AMP.

U.S. Patent 4.223.090 describes reagents for the enzymatic determination of triglycerides. These reagents consist of a lipase, which hydrolyzes the triglycerides to produce glycerol: glycerol dehydrogenase, buffer and NAD, which oxidize the glycerol to produce NADH; and diaphorase and a tetrazolium salt, which uses the NADH to produce a colored formazan dye and regenerate NAD. The improvement described in this patent is the inclusion of manganese ion which is present at concentrations from 0.05 to 0.15 mM.

U.S. Patent 4.215.197 describes the test means and method for creatine determination. In this patent. creatinine is enzymatically hydrolyzed to creatine. This creatinine is further enzymatically hydrolyzed to sarcosine and urea, and the sarcosine is enzymatically coverted to formaldehyde and glycine with the production of NADH. The NADH then reduces the tetrazolium salt, MTT, either directly or via diaphorase to produce a colored dye. The patent also describes the production of a dried reagent tablet containing the described ingredients.

U.S. Patent 4.142.938 describes a method for the determination of triglycerides and glycerol. In this patent, triglycerides are hydrolyzed by sodium hydroxide treatment at moderate heat. The resulting glycerol is then removed from interfering substances by treatment with magnesium ions, which precipitates the interfering substances. The sample is contrifuged to remove the precipitate and the supernatant is assayed for glycerol content in a liquid enzymatic reaction containing: ATP, glycerol kinase, glycerol-1-phosphate dehydrogenase, NAD, diaphorase, and a tetrazolium salt. The glycerol is catalyzed by glycerolkinase. The glycerol-1-phosphate is then oxidized to form NADH and glyceraldehyde-3-phosphate. The NADH is then used to reduce the tetrazolium salt in a diaphorase catalyzed reaction.

U.S. Patent 4,024,021 describes a method for the determination of glutamate and glutamic transaminases in biological fluids. In this patent, substrates for the transaminases are incubated with the transaminases so that glutamate will be produced. The glutamate produced is oxidized by glutamate dehydrogenase in the presence of NAD to produce alpha-oxoglutarate, ammonia, and NADH. The NADH is then reacted with the tetrazolium salt, INT, to produce a colored formazan. This last reaction can be catalyzed either by diaphorase or by the electron carrier, N-Methylphenazine methosulfate. The intensity of the formazan color produced per unit time is measured to give a measurement of the transaminase activity.

U.S. Patents 3,867,259 and 3,867,258 describe the production of lactate dehydrogenase test material. The test material consists of a bibulous material containing the dried residue resulting from the impregnation of the material with a tetrazolium salt, a chromatographic effect preventor, an antioxidant, diaphorase. NAD. and an alkali lactate salt mixture. Patent 3,867,258 discloses the use of diaphorase immobilized to a hydrophilic, cross-linked. sulfated aldehyde or ketone polymer dispersed throughout the interstices of the material. In these patents, lactate dehydrogenase from sera oxidizes the lactate to pyruvate with the concomitant conversion of NAD to NADH. The diaphorase in the material then catalyzes the NADH dependent reduction of the tetrazolium salt to form a colored dye. As diaphorase is present in excess over the lactate dehydrogenase to be assayed, the rate of color formation (the amount of color produced in a unit time) is an indicator of the lactate dehydrogenase concentration in the sera sample.

U.S. Patent 3.791.931 also discusses a reagent and method for the determination of lactate dehydrogenase. This invention uses pig heart diaphorase obtained from the protein fraction of a pig's heart insoluble in 1.6 to 2.8 M ammonium sulfate by treating the insoluble protein fraction with 0.1-0 3% w·v polyethylenimine, heating at 70 to 80 degrees C. absorption on a weakly acidic cation exchanger and subsequent elution. This patent also discusses the use of a buffer, a stabilizer, and bovine serum albumin (BSA) in the aqueous assay of lactate dehydrogenase.

The shortcoming that existed in all of the art prior to the co-pending application is that one equivalent of dye molecule is produced for every biological molecule that is oxidized Quantity of analyte could only be determined by the intensity of the color change. This limitation prevented the assay of "high" concentrations of these biological molecules, as the amount of color that would be generated by the complete or near complete oxidation of such a "high' concentration, in accordance with conventional methods. would yield colored solutions that had too high an absorbance to be read without dilution Additionally. the "high" concentration of dye would lead to solutions wherein dye-dye interaction would cause serious deviation from the ideal as predicted by Beer's law, resulting in solutions that do not have linear relationship of absorbance versus concentration of dye. These "high" concentrations (too high to measure), include the normal concentration as well as the super normal concentration indicative of a disease state of most, if not all, medically important biological metabolites. Therefore, when complete or near complete oxidation of biological molecule was used in the assay methodology common to all previous assays, a massive dilution of the medical sample was necessary to lower the concentration of the biological molecule.

The co-pending patent application overcame these problems. In these inventions less than one molecule of dye is created per molecule of analyte, preventing the occurrence of a color change unless the analyte is present in excess of a predetermined concentration. By testing for a variety of predetermined concentrations of analyte, the analyte concentration may be accurately measured.

Co-pending patent application No. EP-A-279 988 accomplished this by reacting the analyte with a dehydrogenase enzyme specific for that molecule and NAD(P), generating NAD(P)H. NAD(P)H was then used to reduce a chromogen in a diaphorase catalyzed reaction. A competing substrate for the diaphorase catalyzed NAD(P)H reduction was also used to prevent the chromogen from being reduced and changing color until the competing substrate had been consumed.

The invention utilized concentrations of the competing substrate corresponding to predetermined levels of the substrate to be measured. If the substrate concentration did not exceed this predetermined concentration, no color change occurred. Various concentrations of competing substrate were combined to determine the concentration of the unknown to be measured.

Co-pending patent application No. EP-A-279 988 disclosed new classes of chromogens and competing substrates that did not require a diaphorase catalyst to promote reduction of NAD(P)H.

The present invention makes use of oxidase enzyme; oxidase is the generic name for any enzyme that oxidizes a substrate for which it is specific to produce hydrogen peroxide. This eliminates the ingredient NAD(P) used in the prior applications to generate the NAD(P)H to be measured. The oxidases and oxidase substrates of this invention directly generate hydrogen peroxide for measurement.

This invention discloses entire new classes of enzymes, substrates to be measured, chromogens and competing substrates. The invention improves over the prior art because the oxidase enzymes used to directly generate hydrogen peroxide for measurement are as a rule more commercially available and therefore more economical than their dehydrogenase counterparts. Bilirubin oxidase, cholesterol oxidase and glucose oxidase each are more readily available than their respective dehydrogenases. Other such examples are obvious to one of ordinary skill in the art.

The colorimetric assay of hydrogen peroxide, particularly hydrogen peroxide produced by the enzymatic oxidation of organic and biological molecules, is well known in both the scientific and patent literature. U.S. Patent 4,642,286 discusses an assay system for the detection of enthanol, using alcohol oxidase, a peroxide detecting system using peroxidase, and alcohol oxidase stabilizers. The peroxidase color system uses ortho-toluidine as a chromogen. In this system, one molecule of dye is produced for every molecule of hydrogen peroxide.

U.S. Patent 4,414,326 describes an assay system for the detection of cholesterol. In this system, cholesterol is oxidized by cholesterol oxidase to produce an equivalent of hydrogen peroxide. The hydrogen peroxide is used in a peroxidase catalyzed reaction to oxidize 4-amino-antipyrine, which then couples with a phenol to form a dye. Once again, one molecule of dye is produced for every molecule of hydrogen peroxide that is produced in this system. This same basic system is utilized in U.S. Patents 4,378.429 and 3,884,764.

This hydrogen peroxide/peroxidase system has also been extensively utilized in the assay and detection of glucose. For example, see U.S. Patents 4,098,574, 4,211,845, 4,273,868, 4,281,062 4,298,574. 4,211,845, 4,273,868, 4,281,062, 4,298,688, and 4,353,984. U.S. patents 4,166,763 and 4,251,629 use this same hydrogen peroxide peroxidase system for the assay of lactic acid and hydrogen peroxide. respectively.

U.S. Patents 3,886,045 and Re. 29,498 assay in a different manner the hydrogen peroxide produced by the enzymatic oxidation of organic and biological molecules. In these patents oxidase does not directly oxidize the chromogen. Instead, the hydrogen peroxide/peroxidase is used to oxidize a ferrocyanide salt to a ferricyanide salt. The ferricyanide then oxidizes a phenol to a colored quinone in a non-enzymatic reaction.

All of the above-discussed oxidase patents, however. share the same shortcoming of the other prior art, namely, each produce one molecule of dye per molecule of hydrogen peroxide produced in or present in the assay system. Quantitative measurement of analyte concentration could only be made by evaluation of color change intensity. Typical levels of medically significant biological metabolites produce concentrations of reduced chromogen too high to obtain an accurate measurement of metabolite concentration. Massive dilution of the sample to be measured is necessary to obtain an accurate reading.

Attempts by the prior art to alleviate this shortcoming have proved less than satisfactory. European Patent No. WO 85 01747 describes a device for the detection of an analyte in the fluid that produces a digital signal from an analog input. One embodiment of this patent is a series of paper circles with different sensitivities for glucose. Different zones would turn from an off-white color to a reddish brown color at different concentrations of glucose.

A similar system for the direct colorimetric quantitative analysis of hydrogen peroxide is provided by this invention. however, the present invention works differently than does this patent and overcomes some of the serious disadvantages of this patent.

In the European patent, an aqueous sample is applied to a dried reagent formula containing an enzyme that will oxidize the analyte to produce hydrogen peroxide. The reagent also contains a second enzyme and a substrate for this enzyme. When this substrate is hydrated in the aqueous solution, it is brought to the second enzyme where it is reacted upon to form cyanide. Cyanide is an inactivator of the first oxidase enzyme, therefore the reaction is shut down. The hydrogen peroxide that is produced before the reaction of the first enzyme is turned off, is converted to color in a peroxidase catalyzed reaction. or is reacted with ascorbic acid to form colorless dihydroascorbic acid.

Two patents by Hochstrasser, U.S. Patents 4,059,407 and 3,964,871 also address the direct colorimetric quantitative analysis of hydrogen peroxide. No. 3,964,871 describes a device for the detection of glucose. In this patent. a device containing various strips of paper is saturated with a test fluid. The different paper strips all contain glucose oxidase, peroxidase, and chromogen. They also contain different concentrations of an antagonist compound. All antagonist compounds disclosed in this patent are derivatives of catechol.

No. 4,059.407 similarly teaches an oxidase enzyme and chromogen combined with different concentrations of a titrant that reduces both hydrogen peroxide and the chromogen.

Several serious problems exist in the above approaches. In the European patent the cyanide produced by the second enzyme will exponentially inhibit the first enzyme and standardization curves must be run every time a measurement is conducted. Furthermore, the rate of the enzymatic reactions is only proportional to the concentration of substrate when the concentration of substrate is well below the $k_m$ of the first enzyme. The $k_m$ of most enzymes of analytical importance, except for glucose oxidase, lies below the normal physicological concentration of substrate. Therefore, a biological sample will need to be diluted before it can be measured using this technology.

The catechols in U.S. Patent 3,964,871 are well known not to be stable to air oxidation. limiting the reliability of methods and devices disclosed in this patent. Both this patent and No. 4.059,407 teach the use of filter paper manufactured to absorb a set and reproducible amount of liquid as a support member. For an accurate reading to be made, a known quantity of fluid must be sampled. To accomplish this with filter paper is commercially not feasible. However, a bibulous, or highly absorbent, material is required. because oxygen from the air must be introduced as a source of oxygen for the oxidase enzyme to oxidize the analyte to produce hydrogen peroxide. The concentration of medically important metabolites is often found to be in the 1 to 50 millimolar (mM) concentration range. The concentration of oxygen dissolved in air saturated water is only 0.8 mM.

Another problem suffered by No. 4,059,407 is that there is no requirement that the titrant have a larger reduction potential than the indicator. An indicator with a larger reduction potential than the titrant not only will reduce hydrogen peroxide and change color before the titrant is consumed. it will also reduce the titrant and change color. This will produce highly inaccurate test results.

The present invention overcomes these problems by using an air-stable reductant of hydrogen peroxide that has a larger reduction potential than the chromogen. An oxygen porous polymeric mechanical support member that can be mass produced overcomes the shortcomings of using filter paper as a support member.

## Objects of the preferred embodiments of the Invention

There are numerous objects of the invention. One object is to provide an accurate. precise, fast and reliable device which permits quantitative analysis of selected organic molecules normally present in organic liquids or fluids.

Another object is to provide a digital color signal giving an "off"-"on" signal for sought concentration of the unknown.

Another object is to provide an analog to digital chemical color signal device.

Another object is to provide a disposable kit which will give a distinctive color at a threshold concentration of the organic molecule concentration which is to be determined.

Other objects will become apparent to one of average skill in the art in the further description of the invention.

Another object is to provide a system. a method and a device which has numerous practical applications and will contribute to the advancement of technology in the field to which the invention applies

Another object of the invention is to provide a system, a method and a device for the quantitative determination of the concentration of hydrogen peroxide. which may be generated in situ in the presence of appropriate reactants.

Another object of the invention is to provide a system, a method and a device for the quantitative determination of the concentration of an organic molecule which will ultimately generate hydrogen peroxide in the presence of appropriate reactants.

Detailed Description of preferred embodiments of the Invention

The invention is useful in the colorimetric quantitative determination of the concentration of hydrogen peroxide. This invention is also useful in the colorimetric quantitative determination of concentrations of analyte oxidized by oxidase enzymes to produce hydrogen peroxide, which analytes bear a set relationship to the hydrogen peroxide concentration, as will be further disclosed below.

This invention includes a chemical component and a mechanical component. The chemical component produces a digital signal from an analog input. Different regions of the device will generally be set with a different threshold for producing this signal. For example. a device for the measurement of cholesterol could have a plurality of different regions which would respond to increasing concentrations of cholesterol. e.g.. 200, 220, 230, 240, 250, 260, 270, 280, 290, 300, 310, 320, and 350 mg/dl. If a blood sample had a cholesterol concentration of 245 mg/dl, the first four regions of the test device would turn color while the other regions would remain in a starting condition. The detailed working of the chemical component is disclosed below.

The mechanical component of the device provides for precise and reproducible delivery of a set volume of the sample to the measuring region of the device. This precise measurement is conducted without the use of machinery, such as automatic pipettes, or controlled delivery devices.

The chemical component is situated in the mechanical component.

The colorimetric quantitative assay system of the invention comprises a prodye, or chromogen, which is an electron donor, or reductant, of hydrogen peroxide and which is capable of changing color in the visible range. A typical chromogen is azinobis (3-ethyl-benthiazoline sulfonic acid) (ABTS). In the system of the invention the chromogen may be colorless to start with and develop a color upon oxidation, or the compound may be colored to start with and become colorless as the reaction is completed, or the chromogen may be a light color and change to a deep or other distinctive color.

The system of the invention also includes hydrogen peroxide, which may be generated in situ as described hereafter; a first reductant of hydrogen peroxide which is capable of donating electrons to, or reducing, oxidized chromogen and/or hydrogen peroxide; and optionally an electron carrier catalyst that is capable of transferring electrons from the chromogen or the first reductant to hydrogen peroxide.

The first reductant used in the invention is capable of preventing the accumulation of oxidized chromogen and thus the development of color attributable to the oxidized chromogen, causing it to revert virtually instantaneously to or causing it to remain in its unoxidized state. Thus, the first reductant, in accordance with the invention. is capable of regenerating the chromogen. These conditions prevail until the reactant is exhausted.

Thereafter, and in the presence of hydrogen peroxide, the full color of the oxidized chromogen will develop virtually instantaneously. Because of the nature of the reaction, the presence of essentially a trace amount of chromogen will generate color, for practical purposes approximately 1 mM. The concentration of first reductant with respect to the chromogen is not critical and may for instance be 50 100, 200 300 mM or more. When there is still hydrogen peroxide present and the first reductant is depleted, i.e., an excess of hydrogen peroxide is present in the system over the amount of electron-donating first reductant, color will instantaneously develop. For purposes of definition, this is the "threshold" amount of hydrogen peroxide.

Thus the system gives a digital reading of two states, and two states only: an "off" and "on" state, the first corresponding to no "color". the second to "color". By "on" and "off", there is meant as described above a "change of color" not only "color" to "colorless" or vice-versa. Thus, unlike an embodiment of the earlier referred to patent application (and unlike many conventional prior art methods and systems), in the present invention the concentration of the hydrogen peroxide does not correspond to an intensity of color generated. nor is it necessary to perform any plotting (color generated vs. standard color curve) to know the concentration of the hydrogen peroxide or of the organic compound (or substrate) which generated the hydrogen peroxide, when such substrate is present.

In accordance with the teaching of the present invention, it will be seen that in cases where the color is in an "on" state there may be and there is generally likely to be present an excess of hydrogen peroxide in excess of the trace, or as stated above about 1 mM chromogen necessary to develop the color upon depletion of the first reductant. As an illustration, in a system which at starting conditions contains 20 mM of first reductant, 1 mM of chromogen and 40 mM of hydrogen peroxide there results (in the situation where 1 mole of first reductant reactant donates 2 electrons per molecule to 1 mole of hydrogen peroxide), 20 mM of depleted first reductant, 1 mM of oxidized chromogen and 19 mM of excess hydrogen peroxide. This excess of hydrogen peroxide does not con vey relevant information for the purpose of the invention.

The system will have given an "on" reading at the instant when and by the development of strong color when a trace or in this instance approximately 1 mM of oxidized chromogen will have been formed by reaction with the 1 mM of hydrogen peroxide.

Also in accordance with the invention, it will be appreciated that the invention is not limited to absolute numbers, as illustrated above, for instance, for the 1 mM. What is more significant is the color formation attributable to the oxidized chromogen or dye. Ideally, the extinction coefficient of the oxidized chromogen is so high that less than 1 mM of oxidized chromogen is required to cause an abrupt and strong positive color development or signal. The extinction coefficient of oxidized chromogens are known and when not known or readily available, can be readily determined by one of average skill in the art without undue experimentation. Ideally, therefore, a chromogen is to be selected which in the oxidized state gives a "clear" sharp, unequivocal, distinct color change in the system of the invention upon reaction of the hydrogen peroxide.

In the situation where the first reductant is not depleted when hydrogen peroxide is depleted, it is evident that chromogen will still be present but no excess oxi dized chromogen will be formed and hence no color change occurs.

There is an important aspect of the invention which will become more apparent from the discussion which follows.

In accordance with the invention, it has been discovered that the concentration of hydrogen peroxide can be measured very accurately and efficiently in an "on-off" digital manner. Where the first reductant is oxidized until essentially depleted, a visible color develops if and only if excess hydrogen peroxide is present; if none is present, no color develops. Thus, in accordance with the invention, a particular pre-selected concentration of first reductant corresponds to a threshold accumulation of hydrogen peroxide. If it is above the threshold, a color will develop when the first reductant is exhausted, but no color, when the first reductant is not exhausted and the hydrogen peroxide is below the threshold. In accordance with the invention, several concentrations of the first reductant are pre-selected and the development of color corresponds to concentration of the hydrogen peroxide in the system to be determined, whether it is a liquid or preferably, a device with a solid support for the reactants.

The determined concentration of hydrogen peroxide also corresponds to the concentration of the organic compound which is sought to be determined, if one is present and which generates hydrogen peroxide (in the presence of the appropriate oxidase).

At the threshold, the concentration of first reductant is equal to 1 or 2 times that of hydrogen peroxide, this being dependent on whether the first reductant is capable of donating 2 or 1 electron to the hydrogen peroxide. Where the first reductant is capable of donating 2 electrons, the relationship is 1 to 1. and it is 2 to 1 when the first reductant is capable of donating 1 electron to the hydrogen peroxide.

In addition to the system of the invention, and various devices, the invention provides a method for measuring hydrogen peroxide which may be generated in situ by an organic substrate the concentration of which is sought to be determined. In that embodiment. an appropriate oxidase specific for the substrate is also present, as will be described further hereinafter.

The process of the invention comprises bringing together the necessary components of the system. The components or reactants are conveniently in an appropriate medium such as a liquid (e.g. aqueous) medium generally for most practical embodiments in or on an appropriate physical support. Such physical supports are known in the literature and are described in the patent application referenced herein above.

There are a wide variety of suitable physical supports that can be used in this invention. In general, the support member may be any material capable of bearing the system for exposure to the liquid solution to be measured. preferably the support is inert enough to the reactants (and products) not to interfer adversely with the reaction. Specific examples of support materials are sheets. rods. webs. filters. strips and similar forms of glass, cellulose, wood, metal, or polymerics such as polyethylene, polypropylene, polyalkyleneacetate, polycarbonates, polymeric fluorocarbons, and the like, or textiles and the like. In one embodiment of the invention, the system can be incorporated into a bibulous material that is capable of taking up, by hydration or capillary action or other similar means, a set and reproducible amount of liquid. Materials that meet these criteria can be manufactured from a wide variety of solids. Examples of solid supports are: metal oxide, as typified by Norton Corporation's (Worcester, MA) controlled pore alumina HSA catalytic rings; polymeric materials, as typified by Nucleopore controlled-pore polycarbonate membranes; and hybrid ceramic/polymer materials as typified by Amerace's polyvinyl-chloride sheets that contain embedded silica particles. Such hybrid sheets are described in U.S. patent 4,169,014, which describes the coupling of active enzymes to these sheets. This patent does not address the utility of these sheets containing said active enzymes in containing a known and calibrated volume of liquid material.

In the capillary embodiment, the chemical components or reactant materials will ideally be deposited as ingredients of thin, water swellable films, such as gelatin films. These films serve to rapidly hydrate in the presence of aqueous sample, presenting the chemical reagents to the sample while simultaneously holding any dye that is formed in place.

When aqueous sample is introduced into the capillary, the gel, which may be made of any organic or synthetic polymer, like: gelatin, agarose, agar, polyvinyl alcohol, polyvinyl pyrrolidone, alginate, carrageenan, dextran, xanthan gum, or mixtures of the above, swells and rehydrates, activating the dried system ingredients. A non-obvious advantage of this system is that multiple layers of the gelatin films may be arranged within the mechanical component, with different layers holding different components of the invention. The use of different layers permits the storage of different components of the invention under different conditions chosen especially for the stability of the component. For example, the enzyme component of the invention may show stability at a pH or ionic strength that is different than that is optimal for the reaction of the components. Should this be the case, a thin film layer can contain the enzyme(s) component under different conditions than those of other layers. Thus, storage incompatabilities of system ingredients is not encountered in multilayer systems.

The invention is not limited to the particular physical embodiment described above. There are numerous appropriate physical arrangements that are described in the literature and others can be built by one of average skill in the art.

In accordance with the invention, the ingredients of the system are brought together as a reaction mixture which contains the chromogen, the electron-donating reductant and the catalyst. To the mixture there is added hydrogen peroxide until a color change occurs. The color change is indicative of the exhaustion of the first reductant and that chromogen has been oxidized to a strong visible color.

In accordance with the method, various linearly related concentrations of the first reductant can be provided in different regions of an assay device. For example, linearly related differing first reductant concentrations can be situated along the "X" axis of a device containing this system. Thus, an "on" signal will be generated at a different linearly related concentration of hydrogen peroxide or substance that generates hydrogen peroxide along the "X" axis. When the first reductant is situated in this matter, increasing either linearly along the "X" axis or in steps of increasing or decreasing concentration, the amount of hydrogen peroxide that is introduced into the system to effect measurement, will be determined by visual inspection of the distance that "on" signal, that is to say, color change, is propagated along the "X" axis.

When in accordance with the invention it is desired to measure the concentration of an organic substrate, the method comprises bringing together the chromogen, the electron-donating first reductant, the catalyst, and a suitable specific oxidase for the organic substrate to be measured. To this reaction mixture there is added in increasing amounts of the substrate to be measured, causing the substrate to be oxidized, producing hydrogen peroxide in proportion to substrate concentration and the reaction to proceed until color is generated, as described above. At that point the first reductant is depleted and hydrogen peroxide generated from the substrate reacts with chromogen to yield oxidized chromogen and a highly visible color change indicative of the concentration of the substrate.

Generally the reaction is carried out in a buffered environment at a pH preferably optimum for the catalyst and enzyme used, as is described hereinafter and at optimum temperature.

The reaction may be carried out in a liquid or on a suitable physical carrier which allows for the reactants to react as described.

Although the invention is not limited by any particular scientific theory or principle, the invention can be considered as having three principal embodiments.

In the first embodiment, the first reductant reacts with the oxidized chromogen as it is produced by the catalytic oxidation of the chromogen and thus prevents the formation of colored chromogen. When the first reductant is all consumed as discussed above, colored oxidized chromogen is then generated catalytically. where color is indicative of the presence of the organic molecule, the concentration of which is sought to be determined.

In the second embodiment, it appears that the first reductant will donate electrons directly to. and thus react directly and preferentially with, the hydrogen peroxide in the system. or as it is formed from the organic molecule. When it is exhausted. the hydrogen peroxide will react with the chromogen forming oxidized chromogen. as described above.

In the third embodiment, the first reductant simultaneously reacts with and reduces both hyrogen peroxide and any oxidized chromogen that may have formed by reacting with hydrogen peroxide, thereby preventing an observable color change until the second reductant has been consumed.

It is not essential that a distinction between the three embodiments be made in the practice of the invention since in all cases color produced by the oxidized chromogen readable (without dilution) in the visible range will be formed.

It will have become apparent from the discussion of the invention that where the threshold is reached - and an "on" signal is read - there is produced less than 1 equivalent of dye (oxidized chromogen) per equivalent of hydrogen peroxide at any concentration of hydrogen peroxide, this being another distinction over the prior art.

As a result of research connected with the development of this dual reductant system, the definitional requirements for an ideal first or competing reductant of hydrogen peroxide for the purpose of a main embodiment of the invention were discovered. A first reductant is, in accordance with these findings. a compound which meets a four-way test.

The first requirement for the first reductant is that, in accordance with the invention. it be capable of reducing hydrogen peroxide in an uncatalyzed or peroxidase catalyzed reaction. This feature is critical to the invention. Without it, the prevention of any detectable color change by the chromogen. unless a threshold concentration level of analyte is present, will not occur. This property can be determined as follows.

A measured solution of hydrogen peroxide, approximately 10 mM. is made up in 10 mM pH 7.4 phosphate buffer, a measured volume of a concentrated first reductant solution is added to this solution so that the final solution of first reductant will be between 5 and 10 mM. 10 IU/ml peroxidase are added to a duplicate reaction and the two are incubated for one hour at room temperature. A first reductant passes this test if. at the end of the incubation, the first reductant is substantially consumed, less than 1 mM remaining, and there has been a corresponding decrease in the concentration of hydrogen peroxide. This test is additionally informative in two different matters. It yields information as to if the first reductant is a substrate for peroxidase enzyme and/or is capable of directly interacting with and reducing hydrogen peroxide. Measurement of both the amount of first reductant and hydrogen peroxide consumed in the incubation provides information as to the electron donating capability of the first reductant. It is well known that the reduction of hydrogen peroxide to water requires two electrons. Reductants are generally capable of donating either one or two electrons. Thus, if an equal amount of hydrogen peroxide is consumed for each equivalent of first reductant, the first reductant is a two electron reducer. Alternatively, if one-half equivalent of hydrogen peroxide is consumed, then the first reductant is a one electron donor.

The second requirement for suitable first reductant in accordance with the invention is that it be stable to air oxidation. Any decrease in the concentration of said first reductant from oxygen degradation will lower the analyte concentration effective to cause a color change in this invention, rendering the reading in error. This property is determined as follows.

A 50 ml solution of the compound to be tested containing an exact known concentration of compound of approximately 50 mM, is put into a 100 ml graduated cylinder. A plastic tube is placed in the cylinder. and a steady air stream is bubbled through the solution for 30 minutes. The air is turned off. and the concentration of unreacted first reductant is determined chemically. The first reductant is said to pass this test if greater than about 99.5% of the first reductant remains in an unreacted state.

Compounds that can be used as a first reductant in this invention but which failed this air stability test include organic reductants containing 1,2-diols and ascorbic acid and its salts. It is well known that ascorbate is not stable to oxidation by atmospheric oxygen. The same disadvantage of sensitivity to air oxidation is seen in the aromatic molecules containing the 1,2-dihydroxybenzene moiety found in catechols disclosed as electron donating molecules by Hochstrasser in U.S. Patent 3,964,871. The molecules, such as 3-methoxy-4-hydroxymandelic acid, 3,4-dihydroxyphenylacetic acid. DOPA. dopamine and adrenaline. are well known to suffer from extreme air instability.

Compounds were discovered that did pass this air stability test. namely ferrocyanide, nitrite. and stable complexes of ferrous iron. for example. ferrous citrate, ferrous EDTA chelate and the complex of ferrous ion with ethylenediamine. An inspection of published values of electronegativity, measured as $E^O$, gave an explanation of these observations.

The reductants that passed this test have a more positive oxidation voltage than does the hydrogen peroxide/oxygen couple (See Table 1). Thus, oxidation of these molecules by two electron reduction of molecular oxygen is expected to be an energetic uphill reaction. Other explanations for the observed air stability are possible, for example. a kinetic barrier to the reaction between reductant and molecular oxygen. Additional molecules that act as air stable reductants may be discovered by use of the first test disclosed above, which will yield yet additional reasons for their observed air stability. The scientific explanation or hypotheses proposed are not critical to this invention. The above described test adequately tests for air stability and can be employed by anyone with average skill in the art.

TABLE 1

| System | $E^O$ (pH 7), volts |
|---|---|
| Oxygen/water | 0.815 |
| Ferric/ferrous | 0.77 |
| Nitrate/nitrite | 0.42 |
| Ferricyanide/ferrocyanide | 0.36 |
| Oxygen/hydrogen peroxide | 0.30 |
| Cytochrome $a$; ferric/ferrous | 0.29 |
| Cytochrome $c$; ferric/ferrous | 0.22 |
| Crotonyl-SCoA/butyryl-SCoA | 0.19 |
| Methemoglobin/hemoglobin | 0.17 |
| Cytochrome $b_1$; ferric/ferrous | 0.12 |
| Ubiquinone; ox/red | 0.10 |
| Dehydroascorbic acid/ascorbic acid | 0.08 |
| Metmyoglobin/myoglobin | 0.046 |
| Fumaric acid/succinic acid | 0.03 |
| Methylene blue, ox/red | 0.01 |
| Yellow enzyme, $FMN/FMNH_2$ | -0.122 |
| Pyruvate + ammonium/alanine | -0.13 |
| $\alpha$-Ketoglutarate + ammonium/glutamic acid | -0.14 |
| Oxaloacetate/malate | -0.17 |
| Pyruvate/lactate | -0.19 |
| Acetaldehyde/ethanol | -0.20 |
| Riboflavin, ox/red | -0.21 |
| Glutathione, ox/red | -0.23 |
| Acetoacetate/$\beta$-hydroxybutyrate | -0.27 |
| Lipoic acid, ox/red | -0.29 |
| $NAD^+$/NADH | -0.32 |
| Pyruvate/malate | -0.33 |
| Uric acid/xanthine | -0.36 |
| Carbon dioxide/formate | -0.42 |
| $H^+$/$H_2$ | -0.42 |
| Acetate/acetaldehyde | -0.60 |
| Succinate/$\alpha$-ketoglutarate | -0.67 |
| Acetate + carbon dioxide/pyruvate | -0.70 |

Oxidation-reduction Tables, such as the above table published in Malher and Cordes, Biological Chemistry, (Harper & Row, New York, 1966), and Hampel, Encyclopedia of Electrochemistry, (Reinhold Publishing Corp., New York 1964) are incorporated herein by reference.

First reductants that passed the air stability test were subjected to the test of their ability to reduce hydrogen peroxide. Surprisingly, sodium or potassium nitrite did not reduce hydrogen peroxide in either an uncatalyzed or peroxidase catalyzed test. This is additionally surprising because sodium nitrite is disclosed is a reducing agent in Hochstrasser U.S. Patent No. 4.059,407.

Ferrocyanide was demonstrated by the above tests to be an ideal candidate reductant. Ferrocyanide

efficiently reduces hydrogen peroxide to water in a peroxidase catalyzed manner.

The third requirement for a suitable reductant candidate is that the oxidized first reductant and or the first reductant must not interact with hydrogen peroxide to produce active hydroxyl radicals with resulting denaturation of enzyme activity, as with aqueous complexes of ferrous ion.

It is well known that ionic iron will interact with hydrogen peroxide to yield a very active reducing species, known as "Fenton's" reagent. This reagent has been known to be a source of hydroxide radical. The hydroxide radical so formed is capable of extracting an electron from many sources in solutions in a non-controlled manner, for example, amine containing buffers, and any enzymes which may be present in the solution, leading to enzyme denaturation.

Ferrocyanide, on the other hand, remains an ideal first reductant candidate. The product of the reaction, ferrocyanide, is a stable ion that does not interact with hydrogen peroxide and will not lead to hydroxide radical formation.

The fourth requirement for a suitable first reductant is that the first reductant have a larger reduction potential than that of the chromogen. Said reduction potentials can also be obtained by reference to Electronegativity tables published in Malher and Cordes, Biological Chemistry, and Hampel, Encyclopedia of Electrochemistry, supra.

As discussed above, unless the first reductant has a larger reduction potential than the chromogen, not only will the chromogen reduce hydrogen peroxide and change color before the first reductant is consumed, the chromogen will also reduce the first reductant and change color.

Otherwise, the choice of the electron donating reductant is wide within the above-disclosed and certain other important limitations. Namely, the first reductant should be inert with respect to the oxidase enzyme or the catalyst. In addition, the first reductant should be colorless with respect to both the oxidase enzyme and the catalyst in the absence of hydrogen peroxide.

It is advantageous to use as a first reductant a water-soluble salt of an element that oxidizes to form a water-soluble salt of the element at a higher valence, and preferably one that possesses the characteristics set forth above. The stable complexes of ferrous iron are one example. Other water-soluble salts of elements that oxidize to a water-soluble salt of the element at a high valence, suitable for use in the assay system of the present invention, are well known to those skilled in the art.

It is by far preferable in accordance with the invention that the first reductant fulfill all of the above requirements. However, under more forgiving situations where it might not be essential for the most accurate results to be obtained, then it may not be necessary that the first reductant pass all of the above tests. It may be acceptable that one or more of the above tests not be fully met. It is contemplated by this invention that one skilled in the art still be within the spirit and the letter of the invention where the first reductant is used within the system described herein.

A first reductant of choice is ferrocyanide, and its alkali metal salts like sodium, potassium, and other equivalent water-soluble iron salts, or other equivalent. Bisulfate and NAD(P)H are also preferred first reductants that have excellent stability to air oxidation. The use of NAD(P)H or ferrocyanide as first reductants has not been disclosed in the previous literature. The use of NAD(P)H or ferrocyanide as first reductants presents additional benefits besides their resistance to air oxidation. Both of these first reductants do not form chelates with other molecules, so that an extremely wide range of other system components can be chosen. However, any air-stable reductant of hydrogen peroxide, which does not interact with hydrogen peroxide to produce active hydroxyl radicals and which has a larger reduction potential than the chromogen in the system, may be used. One skilled in the art is quite capable to identify additional substances which are suitable first reductants because the parameters or tests for such reductants are disclosed herein.

The choice of the electron donating chromogen is not critical to this invention. Any of a wide variety of candidates that have been disclosed in the scientific and patent literature can be equally utilized in this invention. Chromogen such as ABTS, orthodiansidine, 4-aminoantipyrine and 4-aminoantipyrine plus phenol have been successfully utilized and are preferred. In general, any molecule that will donate electrons to hydrogen peroxide in a catalyst catalyzed reaction with a resulting change in visible color can be used in this system, so long as the electron donating strength of the chromogen is less than that of the first reductant. The electron donating strength of the chromogen relative to the first reductant and hydrogen peroxide may be determined as follows.

An equivalent of a candidate chromogen is added to an equivalent of hydrogen peroxide in a flask with a catalytic amount of peroxidase. A candidate chromogen initially passes this test if it changes color in the presence of hydrogen peroxide. The resulting dye is aliquoted into test tubes and candidate first reductants, which should possess the characteristics set forth above, are added to the test tubes in a quantity in excess of one equivalent. A chomogen first reductant combination passes this test if the dye color is removed, i.e.,

the oxidized chromogen has been reverted to chromogen. This indicates that while both the chromogen and the first reductant are reductants of hydrogen peroxide, the first reductant has a higher reduction potential than the chromogen.

A wide variety of compounds are available for choice of the catalyst, as long as these molecules do not react or interact with any other component of the system to produce appreciable visible color in the absence of hydrogen peroxide. One embodiment of this invention uses a peroxidase enzyme as the catalyst. Peroxidase has been widely utilized in the patent and scientific literature for its ability to transfer electrons from an electron donor to hydrogen peroxide, as has been disclosed above. In addition to hydrogen peroxide, certain metal ions can be used as this catalyst. It has been previously disclosed that iron ions can be substituted for peroxidase as the catalyst in this transfer reaction. For example, water soluble derivatives for ferrocene can efficiently catalyze the transfer of electrons from both electron donors to hydrogen peroxide. We have also shown that ferrous ion can efficiently effect this same transfer.

In accordance with the invention, the range of catalysts and first reductants can be greatly enhanced by the presence of added materials in the system. It is advantageous to provide the catalytic electron-transfer in the presence of a salt, which may be preferably an inorganic salt. Of particular interest are organic ligands of metals like ferrocene (dicyclopentadienyliron). The noble metals like platinum or palladium, in catalytic amounts, enhance the catalytic activity of the catalyst. Suitable are water-soluble salts of palladium like palladium chloride.

When NADH was first tried as a first reductant with peroxidase as the catalyst, no reduction in color occurred. It was noted that Brooks, et al (Biochem, Biophys, Res, Comm. 116, 916-921 (1983) showed that horseradish peroxidase will oxidize NAD(P)H, NADH, glutathione and indole-3-acetic acid in the presence of manganous ion. When approximately 2 mM of manganous ion was added to the system described above, NADH, NAD(P)H, glutathione and indole-3-acetic acid became acceptable or excellent first reductants with peroxidase acting as a catalyst. The paper by Brooks does not disclose the use of these substrates to reduce the amount of color that is generated in peroxidase catalyzed reactions. The patents discussed above do not disclose the addition of manganous ion to increase the range of acceptable first reductants.

In accordance with the invention, the system (and the process) of the invention can be used to assay hydrogen peroxide directly. In another embodiment, the invention is useful to assay and determine the amount of hydrogen peroxide generated by any chemical (also enzymatic) or electrochemical method.

The source of the hydrogen peroxide is not important to, or a limiting aspect of this invention. The scope of this invention extends far beyond the specific examples used for purpose of discussion and illustration in the experimental methods section. Virtually the concentration of any organic compound which is a substrate for an hydrogen peroxide - linked oxidase system can be determined in accordance with the invention. Organic compounds of interest include acyl-CoA, sugars such as glucose, lactose and galactose; polyalcohols such as glycerol and sorbitol; triglycerides, cholesterol, and alcohols such as ethanol and methanol; caffeine, xanthine, bilirubin, pyruvate oxalate, ascorbic acid, choline, glycerol phosphate, sarcosine, cytochromes, NADH, and glutothione; amine compounds such as mono-and di-substituted amines, L-and D-amino acids, and other proteins; ketones and organic acids like lactic acid and uric acid; aldehydes like formaldehyde and acetaldehyde; beta-hydroxybutyrate and antigens (like hepatitis B surface antigen, antigen(s) of acquired immune deficiency syndrome, immuno deficiency virus and others); nucleotide sequences, glycerol-3-phosphate, glycine lactate and various other organic substrates that are reactive to enzyme-catalyzed oxidation and others disclosed in the literature.

Commercial applications for this invention include the measurement of environmental pollutants such as aliphatic and aromatic hydrocarbons and chlorinated aliphatic and aromatic hydrocarbons; the measurement of bodily fluid levels of therapeutic drugs such as theaflavine, theobromine, cyclosporin and various antibiotics; and the measurement of bodily fluid levels of illicit drugs such as cocaine, various opiates and various tetrahydrocannabinols (THC'S). The assay of other organic molecules and the exploitation of commercial applications, which would be obvious to one of ordinary skill in the art, is specifically contemplated by this invention.

Commonly the substrate is present in a biological fluid like serum, blood, urine, semen, saliva, cerebrospinal fluid or other liquids of humans or other mammals for instance of other species.

The fluids are not limited to those obtained from humans but also include those obtained from other mammals in general, including, for example, bovine, porcine, equine, feline and canine fluids. The fluids also include those obtained from non-mammals such as fish.

When the substrate is present for the determination of its concentration, the system also includes a specific oxidase for the specific substrate. Oxidases are of course known. Typical oxidase are the following commercially available oxidases or to be available oxidases or any other oxidase can be substituted for the oxidase used in each specific example:

Acyl-CoA oxidase, alcohol oxidase, ascorbate oxidase, bilirubin oxidase, cholesterol oxidase, choline oxidase, glucose oxidase, glycerophosphate oxidase, lactate oxidase, pyruvate oxidase, and sarcosine oxidase are available in bulk from Finnsugar Biochemicals, Inc. (Elk Grove Village, Illinois). In addition, galactose oxidase, glycolate oxidase, oxalate oxidase, L-amino acid oxidase, D-amino acid oxidase, monoamine oxidase, diamine oxidase, cytochrome oxidase, NADH peroxidase, lactoperoxidase, and glutathione peroxidase are available from the Sigma Chemical Company (St. Louis, MO).

It is important to note, that in accordance with the invention, it is readily apparent to those skilled in the art that these oxidases can be used in conjunction with other protein catalysts to assay a wide variety of biological molecules. The compound, the concentration of which is sought to be determined, need not be itself a substrate for the oxidase. It is sufficient that it be capable of, and generate, a substrate for the oxidase. For example, esterified cholesterol can be suitably detected and assayed by use of cholesterol esterase, which produces free cholesterol, and cholesterol oxidase, which oxidizes cholesterol to produce hydrogen peroxide. The use of this assay system in all such combinations, which are readily apparent to those skilled in the art, is specifically contemplated by this invention.

Generally, any enzyme capable of oxidizing a substrate to produce hydrogen peroxide may be used, such as those listed in Barman, Enzyme Handbook, (Springer-Verlag, New York, 1969, Supplement 1974), which is incorporated herein by reference.

The device of the invention, which is often discardable after the threshold color will (or will not) have developed comprises a support member bearing a single or a plurality of concentrations of the first reductant. The support member is not critical in the sense that a specific material of construction is required although several forms of the preferred embodiment will be described hereinafter. In general, the support member may be of any material capable of bearing the reactant and the other components of the system, if necessary, for exposure to the solution to be tested. Specific examples of support members are webs, sticks, strips, splinters, sheets, rods and like forms of glass, metal, wood, paper; polymerics such as polyethylene, polypropylene, polyalkylene acetate, polycarbonates, flurocarbon polymers and like materials, textiles and the like. Preferred materials are the bibulous materials such as microporous tetrafluoropo lyethylene, or microporous teflon, upon which can be deposited layers of gelatin film, each containing a particular reactant. Other preferred bibulous materials are filter paper and blotting papers. Physical systems of the invention have been discussed above.

The reactant components of the assay system of the invention are preferably prepared in a gel form for deposit upon the support member. Once placed on the support member, the reactant compositions in solution are dried to adhere the compositions to the support member. Generally, adhesion of the reactant compositions to the support member is conveniently effected when the support member is a bibulous material. Conventionally employed inert filters, binders, surfactants and the like may be incorporated into the reagent compositions when desired. Certain binders such as resin gums are advantageously incorporated into the reactant compositions to assist in adhering them to non-porous support members such as metal, glass or non-porous polymeric materials. For product elegance and accuracy, it is desirable that the color change in each indicator zone of the devices of the invention be clear, sharp, unequivocal and strongly positive.

The device may be used as disclosed in U.S. patent 4,059,407 by immersion in the biological solution to be tested as for instance in a capillary. When it is desired that the device function in a thermometer-like fashion, the device is adapted to have several sites or regions. The device may be constructed as disclosed in said 4,059,407 patent. Reference is also made to its Figures which illustrate suitable physical embodiments of the present invention.

The following examples are merely illustrative of the invention and of the best mode now contemplated by the inventors presently. The examples are not to be construed as limiting the invention. One skilled in the art without undue experimentation can make various substitutions and variations and by equivalent means, performing in substantially the same manner, obtain substantially the same results without departing from the teaching and spirit of the invention.

All parts are by weight unless indicated otherwise.

Example 1

A disposable device for measuring blood total and direct bilirubin.

On a plastic sheet that will form the bottom of a capillary device is laid down a multilayer gelatin film, one layer containing:

100 IU/ml bilirubin oxidase
20 IU/ml peroxidase
2 mM manganous sulfate
100 mM Tris-chloride buffer, pH 8.2
1 mM ABTS

Another layer of this system contains a horizontal linear gradient of ferrocyanide running from $1 \times 10^{-7}$ moles at one end to $2 \times 10^{-5}$ moles at the opposite end. Additional layers of this device contain polymeric molecules and detergents to assist in the spreading of the liquid sample throughout the capillary. Additional layers can be optionally added as swelling layers to cause the gel to swell to fill the entire capillary upon hydration. The choice of detergent and the location of detergent can be varied to distinguish direct from total bilirubin.

Additional components to this system can be organic anions to displace the bilirubin from its albumin binding sites.

This bottom layer is then sonically welded to a top layer so as to form an exact flat capillary that will contain 10 microliters of solution. This top layer also contains a sample well, which has an absorbative material covered by a membrane to prevent the passage of red blood cells into the capillary measuring region, and an air escape hole to allow the escape of air from the capillary as it is displaced by the serum sample.

The top of the top layer contains a linear scale that exactly coincides with the linear gradient of ferrocyanide in a lower layer. This device as described will measure from 0.3 to 5 mg/dl bilirubin as a colored column. The greater the length of the column, the higher the concentration of bilirubin.

In this device, glucose oxidase, ascorbate oxidase or pyruvate oxidase may be substituted for bilirubin oxidase, with adjustments to amounts used that would be obvious to one skilled in the art, for measuring concentrations of glucose, ascorbate and pyruvates respectively.

## Example 2

A disposable device for the measuring of blood cholesterol.

A blood cholesterol measuring device is constructed similar to the bilirubin device discussed above except that cholesterol oxidase is substituted for bilirubin oxidase. If total cholesterol is to be measured, cholesterol esterase is additionally added to this system. The linear gradient of ferrocyanide is changed to $1 \times 10^{-5}$ moles at the lower end to $4 \times 10^{-4}$ moles at the higher end. This device as described will measure from 1 to 20 mM cholesterol.

This concentration of cholesterol is higher than the concentration of dissolved oxygen, therefore, the multilayer gel film is cast on an oxygen permeable membrane, such as a microporous nylon membrane, instead of directly on the bottom of the device. The plastic bottom of the device contains a multiple of holes in the measuring region to allow the free passage of air oxygen to the microporous membrane.

In this device, acyl-CoA oxidase, galactose oxidase or choline oxidase may be substituted for cholesterol oxidase, with adjustments to amounts used that would be obvious to one skilled in the art, for measuring concentrations of their respective substrates.

## Example 3

A disposable device for the measurement of alcohol in saliva.

A polyvinyl-chloride sheet containing silica particles (Amerace) is made to exact and reproducible thickness. This sheet is then soaked in a solution containing:

22 mM bisulfite
200 mM Tris pH 9.5
2 mM ABTS
0.02 mM ferrous chloride
200 IU/ml alcohol oxidase

and ingredients for the stabilization of the enzyme, then dried in a hot air stream at reduced pressure. This sheet is cut into small sections, the dimensions of which are not critical, and fastened onto a plastic carrier. When this sheet is touched to a solution of saliva, the sample is carried into the polyvinyl-chloride material by capillary action. This sheet will turn turquoise if contacted by samples containing greater than 0.1% alcohol, but will stay light brown gray if it encounters solutions with a less than 0.1% concentration. Thus, this device is an analog to digital colorimetric converter that has great utility in the determination of legal levels of alcohol.

In this device, oxalate oxidase, lactoperoxidase or glutathione peroxidase may be substituted for alcohol oxidase, with adjustments to amounts that would be obvious to one skilled in the art, for measuring concentrations of their respective substrates.

EXAMPLE 4

A disposable device for the detection of serum lactate.

A multilayer gelatin film is cast onto a microporous membrane, one layer of the film containing:

100 IU ml lactate oxidase
0.5 mM ferrocene
1 mM ortho-diansidine
100 mM Tris buffer, pH 8.0,
another layer of the film containing sections 0.1 inch wide each section contains an increasing concentration of ascorbic acid from $5 \times 10^{-5}$ mole to $1 \times 10^{-3}$ mole. Additional layers are spreading and swelling layers as discussed above. This film is cut into sections 1 inch long and 2mm wide. The film section is incorporated into a plastic capillary (as discussed above) with the final volume of the capillary being 50 ul. When serum is introduced into this capillary, a color change will occur between 1 mM lactic acid in the section containing $5 \times 10^{-5}$ and 20 mM in the section with $1 \times 10^{-3}$ mole.

In this device, glycerophosphate oxidase, glycolate oxidase and NAD(P)H peroxidase may be substituted for lactate oxidase, with adjustments to amounts that would be obvious to one skilled in the art, for measuring concentrations of their respective substrates.

Example 5

A method for the commerical mass production of a Digital Colorimetric Quantitative Assay Device.

A roll measuring 1.5 inches wide of microporous tetrafluoropolyethylene (TFPE), commonly known as microporous teflon, is purchased from a variety of sources including DuPont Chemicals or Gore, Inc. This roll is incorporated into a coating machine. The microporous material first passes through a corona discharge station (Station A). In this station, an electrical corona discharge machine, for example a Tautec Corona Generator (distributed by Tampo Print, Schaumburg, IL), temporarily modifies the surface of the normally hydrophobic microporous material, rendering it hydrophilic. In the coating station (Station B), gel layers are successively placed onto the web. The first layer contains enzymes, for the purpose of this example, cholesterol oxidase at 1-100 IU/ml, cholesterol esterase at 0.1-100 IU ml, peroxidase at 10-1,000 IU ml, as well as necessary buffer salts and detergent. The buffer should be ideally chosen so as to contain a primary amine. The second layer is a non-buffered gel layer which serves to separate the enzyme from the other layers. The third layer contains ferrocyanide. Ideally, this layer should contain set, known and different, concentrations of ferrocyanide in different regions of the microporous material. For example, the ferrocyanide should incease in a linear or non-linear manner across the width of the web. The fourth layer is another thin separating layer and the fifth layer is a wicking layer that will rapidly spread material across the surface. This layer is generally composed of a mixture of cellulose, for example, microcrystalline cellulose and detergent. Optionally, a binder, for example, gel, is incorporated into this wicking layer. The wicking layer is, in effect, a very thin piece of paper which has been cast over the underlying gel layers to assist in spreading of the aqueous sample.

Station B of this coating machine is advantageously enclosed in an air chamber which is cooled to 40° C, by the continuous introduction of cold, dry air into this region. This cooling allows the gel layers to gel as formed and prevents mixing of the components of different layers.

The coated microporous material then passes into the drying station (Station C) where the gel is dried by exposure to a dry air steam heated to 40° C. The dried coated material is then wound on a take-up roll for further processing.

The device is assembled as follows:

The coated, microporous material is fed from the take-up roll to a slicing station where it is cut into 1 8 inch wide strips. These strips are fastened to the edges of a polystyrene box, the edges of which have been coated with a thin ( 0.1 ml) adhesive layer. The hollow, polystyrene box, which now contains the coated material covering one open end of the box with the coated side of the microporous material facing outward, is then covered with a top piece. The coated side of the microporous material is introduced into the top piece. The top piece is then welded to the polystyrene box by use of a sonic welder, yielding a complete, finished device which is then packaged.

As disclosed in the parent cases, this invention can also be used with any mixture of enzymes to assay a biological molecule providing one of the resulting products of this mixture or string of enzymes is hydrogen peroxide.

The system of the invention as noted above will normally contain as is known, various buffers compatible with the enzymes, stabilizers (for the enzymes on the resulting dyes), and, if desired, wetting agents. Illustrations are BSA, polyalcohols, mild reducing agents, non-ionic wetting agents. The pH is generally in the range of about 4 to about 11 (being optimized for the different enzymes used). The optimum pH ranges for different oxidase are known.

Thus, one skilled in the art will find it advisable to adjust the environment wherein the enzymes are to be active (be it the filter paper strip, the test tube, or other liquid or solid medium, etc.) at the optimum pH or within or close to the optimum range or value. It should be noted, that in the case of the multilayer device discussed above, all layers do not have to be stored (or do not have to be) at the same pH, which is greatly advantageous in storage shelf life of the ingredients.

In the case where the hydrogen peroxide is generated by the action of a specific oxygenase acting on its specific substrate, it may be advantageous to incorporate into the buffers molecules that will react with and trap, immobilize, etc., the oxidized specific substrate. These compounds are generally amine containing compounds, for example, tris, or glycine that can also be utilized for pH control. Compounds that are especially useful for this trapping function are those of the general class known as alpha-effect amines. Alpha-effect amines are those amine containing compounds that contain an atom that has unpaired electrons adjacent to the amine functional group. Examples of alpha-effect amines that can be advantageously incorporated into this system are hydrazine, N-substituted hydrazines, hydroxylamine, and O-substituted hydroxylamines, especially those O-substituted hydroxylamines that are stable to drying due to their high boiling nature and low vapor pressure. An especially suited compound of this class is carboxymethoxyamine.

Also useful in the practice of the invention will be chemicals that form gels or films that permit storing the essential ingredients in a dry state and rehydrating in the presence of an aqueous solution and controlling color generation. For such known chemicals see U.S. Patent 4,556,634, column 4, which passage is incorporated herein by reference.

For the purpose of this invention, the "threshold" is defined to mean a color change state which is indicative of the concentration of hydrogen peroxide: when the concentration of hydrogen peroxide is less than a threshold, no color exists; when the concentration of hydrogen peroxide is above the threshold, the clear, sharp, unequivocal preferably strong color will be produced. Where the reduced chromogen is colorless, the reverse situation applies.

Prior art provides description of devices, often disposable which may be used in the practice of this invention as such or in a modification of such devices. The reagents and the reactions (and their sequence) of the system of the invention is novel and unobvious. Reference for such devices is made to U.S. Patents Nos. 4,059,407; and the patents listed therein; 3,964,871; U.S. patent 3,992,158 and the later patent referring to this patent describes a device that is particularly useful to this invention. These patents describe a multilayer analytical element that will change color in the presence of an ana lyzed molecule. The technology discussed in these patents yields an analog color signal for an analog concentration in-put. The device and analytical element described can be modified in light of and with knowlede of the present invention to create a digital color signal. U.S. patents 3,485,587 and 3,164,534 also describe a device that could be modified with the invented technology to improve them to register a digital "on/off" type signal.

Another patent which has also been considered in the preparation of this patent application (in addition to those others listed in the parent application) this PCT Publication WO 85/01747, "Device for Rapid Quantitative Analysis of a Fluid," issued April 25, 1985 to Hof. et al of Inomedix Incorporated.

It is to be noted that it is within the scope of the invention to use more than one reactant (inert with respect to each other) which will be sequentially consumed by the hydrogen peroxide in accordance with the invention. Likewise there may be used more than one chromogen. Similarly, the system can be used to determine more than one organic compound (with their respective enzyme systems), which compounds may have different reactivity levels with respect to the other reactants.

It is within the scope of the invention also to use the method of the invention on a continuous basis in a device adapted to feed the reactants to a multiplicity of reaction zones for the reactions to take place and the color change indications to develop.

Such device is likely to be of particular interest for industrial purposes like monitoring the absence, presence or concentration of organic compounds.

It is also within the scope of the invention to utilize it in the determination of the activity of any enzyme, or combination of enzymes, that result in the production of hydrogen peroxide. The amount of an oxidase enzyme can be determined by the rate at which color is produced in a system containing the enzyme substrate, necessary buffer salts, the ingredient of the invention and an unknown amount of oxidase enzyme. The reduction in the amount of color that is generated in the mixture, as opposed to that generated by conventional mixtures, wherein one molecule of dye is produced per molecule of hydrogen peroxide produced, will result in an advantageous greater period of time when the color is in the linear, visible discernible range.

The invention has been described in detail with particular reference to certain preferred embodiments thereof, but it will be understood that variations and modifications and their equivalents can be effected within the spirit and scope of the invention as claimed. The subject matter of the following claims the numbers of which have been struck through is believed to be in the description above but, if not, is nevertheless part of the invention and may be claimed as such.

## Claims

1. A colorimetric assay system which is designed to measure the concentration of hydrogen peroxide by developing a color signal when the hydrogen peroxide is above a threshold concentration and by the absence of color signal when the concentration of hydrogen peroxide is below the threshold, which system comprises
a chromogen capable of reducing hydrogen peroxide and which is capable of changing color in a visible range to yield an oxidized chromogen of a different color, and an air-stable first reductant of hydrogen peroxide that has a larger reduction potential than that of the chromogen, which first reductant prevents accumulation of oxidized chromogen and thereby a visible color change until the amount of first reductant is essentially depleted at which threshold a color is developed when hydrogen peroxide is present, and in the absence of hydrogen peroxide, no color is developed.

2. The assay system of claim 1 wherein the first reductant is capable of reducing hydrogen peroxide, or oxidized chromogen, or both.

3. The assay system of claim 2 wherein the first reductant is capable of reducing oxidized chromogen, and thereby regenerates the chromogen.

4. The assay system of claim 1 wherein the reduction of hydrogen peroxide by either the first reductant or the oxidized chromogen is promoted by a catalyst and optionally a metal salt which enhances the catalytic activity of the catalyst.

5. The assay system of claim 4 wherein the catalyst promoting the reduction of hydrogen peroxide or the oxidized chromogen does not react or interact with any other component of the system to produce appreciable visible color in the absence of hydrogen peroxide.

6. The assay system of claim 5 wherein the catalyst promoting the reduction of hydrogen peroxide or the oxidized chromogen is selected from the group consisting of peroxidase or horseradish peroxidase.

7. The assay system of claim 5 wherein the catalyst promoting the reduction of hydrogen peroxide or the oxidized chromogen is a water soluble derivative of ferrous ion.

8. The assay system of claim 1 wherein the first reductant is bisulfate or a water soluble alkali metal salt of ferrocyanide.

9. The assay system of claim 5 wherein the first reductant is NAD(P)H and the catalyst is a compound capable of accepting electrons from NAD(P)H, such as NAD(P)H peroxidase, diaphorase or an organic polycyclic compound.

10. The assay system of claim 13 wherein the organic polycyclic compound catalyst capable of accepting electrons from NAD(P)H is selected from the group consisting of meldola blue, phenazine methosulfate, N-methylphenazine methosulfate, methylene blue, or anthroquinones substituted with a member selected from the group consisting of methyl-, chloro-, bromo-, amino-, hydroxy-, nitro, methoxy or sulfuric acid.

11. The assay system of claim 4 wherein the metal salt which enhances the catalytic activity of the catalyst is present and is manganous sulfate, or a water soluble salt of platinum or palladium.

12. The assay system of claim 1 wherein the chromogen is azinobis- (3-ethylbenz-thiazoline sulfonic acid) (ABTS), orthodiansidine or 4-aminoantipyrine, or it is 4-aminoantipyrine and phenol.

13. The assay system of claim 1 wherein when a visible color change occurs indicative of the reductant being depleted and of the threshold concentration of hydrogen peroxide, there is generated less than one equivalent of oxidized chromogen per equivalent of hydrogen peroxide.

14. The assay system of claim 1 which comprises an oxidase which corresponds to a specific organic substrate. the concentration of which is to be measured, and/or an organic substrate corresponding to the oxidase, the concentration of which organic substrate is to be measured.

15. The assay system of claim 21 wherein the hydrogen peroxide is generated by a reaction between the organic substrate and the oxidase which corresponds to said organic substrate.

16. The assay system of claim 22 wherein, the organic substrate is selected from the group consisting of cholesterol, glucose, lactic acid, bilirubin or a compound which reacts to generate said substrate in situ, and the oxidase which corresponds to said organic substrate.

17. The assay system of claim 1 wherein the first reductant is present in different pre-selected concentrations, each concentration corresponding to a concentration of hydrogen peroxide to be determined, wherein of the different first reductant concentrations, some will be depleted and some will not be depleted as being shown by the development of a color and being indicative of the determined threshold concentration of hydrogen peroxide.

18. The assay system of claim 24 wherein the first reductant is a water soluble alkali metal salt of ferrocyanide and the chromogen is ABTS.

The assay system of claim 25 which comprises an oxidase which corresponds to a specific organic substrate, the concentration of which is to be measured.

The assay system of claim 26 which comprises an organic substrate corresponding to the oxidase, the The assay system of claim 27 wherein the hydrogen peroxide is generated by a reaction between the organic substrate and the oxidase which corresponds to said organic substrate.

The assay system of claim 28 which comprises an organic substrate selected from the group consisting of cholesterol, glucose, lactic acid, bilirubin or a compound which reacts to generate said substrate in situ, and the oxidase which corresponds to said organic substrate.

19. A diagnostic colorimetric device which is designed to measure the concentration of hydrogen peroxide by developing a color signal when the hydrogen peroxide is above a threshold concentration and by the absence of a color signal when the concentration is below the threshold. which device comprises a physical support means for a chromogen capable of reducing hydrogen peroxide and capable of changing color in a visible range to yield an oxidized chromogen of a different color, for an air-stable first reductant of hydrogen peroxide that has a larger reduction potential than that of the chromogen and for an electron carrier catalyst, which catalyst is capable of transferring electrons from the first reductant to said hydrogen peroxide or said oxidized chromogen.

The device of claim 30 wherein the physical support means is a water absorbing material inert to the reactants.

The device of claim 31 wherein the water absorbing material inert to the reductant is a hybrid ceramic polymer material.

The device of claim 32 wherein the hybrid ceramic/polymer material is polyvinyl chloride sheet that contains embedded silica particles.

The device of claim 33 wherein the polyvinyl chloride sheet is fastened to a plastic carrier.

The device of claim 31 wherein the water absorbing material inert to the reactants is situated inside of a controlled volume capillary.

The device of claim 35 wherein the water absorbing material inert to the reactants is selected from the group consisting of chromogenic paper, a gel, or a synthetic resin.

The device of claim 36 wherein the water absorbing gel is selected from the group consisting of gelatin, agarose, agar, polyvinyl alcohol, polyvinyl pyrrolidone, alginate, carrageenan, dextran, xanthan gum, or mixtures thereof.

The device of claim 37 wherein the water absorbing gel is a multilayer dry gel.

The device of claim 38 wherein the multilayer dry gel comprises four layers consisting of (1) an oxidase corresponding to a specific organic substrate, the concentration of which is to be measured, (2) a separation layer, (3) first reductant and chromogen, and (4) another separation layer.

The device of claim 39 wherein the multilayer dry gel comprises a fifth wicking layer.

The device of claim 39 wherein the first reductant is ferrocyanide and the chromogen is ABTS.

The device of claim 41 wherein the oxidase is selected from the group consisting of glucose oxidase, lactate oxidase or bilirubin oxidase.

EP 0 317 070 A2

The device of claim 41 wherein the oxidase is cholesterol oxidase.

The device of claim 43 which comprises in addition cholesterol esterase.

The device of claim 40 wherein the wicking layer contains microcrystalline cellulose.

The device of claim 35 wherein at least one surface of the controlled volume capillary consists of material selected from the group consisting of teflon, nylon, polyethylene, polypropylene, polyalkylene acetate or polycarbonate.

The device of claim 46 wherein the concentration of organic substrate to be measured exceeds the aqueous solubility of oxygen, the material is microporous.

The device of claim 30 wherein the concentration of first reductant to hydrogen peroxide at the threshold is either 1 to 1 or 2 to 1, depending on the electron-donating ability of the first reductant.

The device of claim 30 wherein the catalyst which is capable of transferring electrons from the first reductant to hydrogen peroxide or oxidized chromogen is diaphorase.

The device of claim 30 which comprises in addition, a metal salt which enhances the catalytic activity of the catalyst.

A diagnostic colorimetric device which is designed to measure the concentration of hydrogen peroxide by developing a color signal when the hydrogen peroxide is above a threshold concentration and by the absence of a color signal when the concentration of hydrogen peroxide is below the threshold, which device comprises a physical support means for chromogen capable of reducing hydrogen peroxide and capable of changing color in a visible range to yield an oxidized chromogen of a different color, for an air-stable first reductant of hydrogen peroxide that has a larger reduction potential than that of the chromogen, and for an electron carrier catalyst, which catalyst is capable of transferring electrons from the first reductant to said hydrogen peroxide or said oxidized chromogen, in which device the physical support means has a plurality of regions, each region having a different concentration of first reductant, which concentration in the respective regions is in a linear relationship, in which device the visible color change takes place in one region, which is indicative and corresponds to the concentration of hydrogen peroxide.

The device of claim 50 wherein the first reductant is ferrocyanide and the chromogen is ABTS.

The device of claim 51 which comprises in addition, an oxidase which corresponds to a specific substrate, the concentration of which is to be measured.

The device of claim 53, which comprises an organic substrate corresponding to the oxidase, the concentration of which organic substrate is to be measured.

The device of claim 54 wherein the substrate is selected from the group consisting of cholesterol, glucose, lactic acid, bilirubin, or a compound which reacts to generate said substrate in situ.

A diagnostic colorimetric device which is designed to measure the concentration of hydrogen peroxide by developing a color signal when the hydrogen peroxide is above a threshold concentration and by the absence of a color signal when the concentration of hydrogen peroxide is below the threshold, which device comprises a physical support means for a chromogen capable of reducing hydrogen peroxide and capable of changing color in a visible range to yield an oxidized chromogen of a different color, for an air-stable first reductant of hydrogen peroxide that has a larger reduction potential than that of the chromogen, and for an electron carrier catalyst, which catalyst is capable of transferring electrons from the first reductant to said hydrogen peroxide or said oxidized chromogen, in which device the physical support means has a plurality of regions, each region having a different concentration of first reductant, which concentration in the respective regions is in a linear relationship, in which device the visible color change takes place in one region, which is indicative and corresponds to the concentration of hydrogen peroxide, and which device includes an oxidase which corresponds to an organic substrate, the concentration of which is to be measured, the reaction between the oxidase and the corresponding organic substrate producing hydrogen peroxide.

The device of claim 56 which comprises an organic substrate corresponding to the oxidase, the concentration of which organic substrate is to be measured.

The assay system of claim 1 wherein the first reductant is essentially all depleted and oxidized, the chromogen is oxidized, having generated a visible color and water, which visible color is different from the color of the system in the absence of the oxidized chromogen and which visible color is indicative of the concentration of hydrogen peroxide above the threshold.

The diagnostic colorimetric device of claim 30 wherein the organic compound the concentration of which has been measured is in the oxidized state, the first reductant is depleted, and the chromogen is in a oxidized and colored state, which color is indicative of the presence of hydrogen peroxide above the threshold concentration.

20

20. An analytical colorimetric digital method of measuring the concentration of hydrogen peroxide generated by an organic substrate the concentration of which is to be measured by developing a color signal when the hydrogen peroxide is above a threshold concentration and by the absence of a color signal when the concentration of hydrogen peroxide is below the threshold, which method comprises, adding to the mixture which comprises a chromogen which is a reductant of hydrogen peroxide and which is capable of changing color in a visible range to yield an oxidized chromogen of a different color, a first reductant of greater reduction potential than the chromogen, which first reductant prevents accummulation of oxidized chromogen and thereby a visible color change until the amount of first reductant is essentially depleted at which threshold a color is developed when hydrogen peroxide is present and in the absence of hydrogen peroxide, no color is developed, an electron-carrier catalyst, which catalyst is capable of transferring electrons from the first reductant to said hydrogen peroxide or said chromogen, and a specific oxidase to a substrate, the concentration of which is to be measured, a substrate the concentration of which is to be measured, reacting the substrate and corresponding oxidase, thereby generating hydrogen peroxide and allowing the reaction to proceed until the first reductant is depleted, at which point a visible color is generated, which is indicative of the concentration of substrate.